# FASCICULE DE BREVET EUROPEEN

(11) **EP 1 583 461 B1**
(45) Date de publication et mention de la délivrance du brevet: **21.01.2009**
(21) Numéro de dépôt: 04702347.8
(22) Date de dépôt: 15.01.2004
(51) Int. Cl.: A61B 1/005

(54) **STRUCTURE LONGITUDINALE ORIENTABLE**
LONGITUDINALE ORIENTIERBARE STRUKTUR
LONGITUDINALLY-STEERABLE STRUCTURE

(30) Priorité: 15.01.2003 FR 0300421
(43) Date de publication de la demande: 12.10.2005
(73) Titulaire: CENTRE NATIONAL DE LA RECHERCHE SCIENTIFIQUE (CNRS), 75794 Paris Cedex 16 (FR); ECOLE NATIONALE SUPERIEURE DE MECANIQUE ET DES MICROTECHNIQUES, 25030 Besançon (FR)
(72) Inventeur: ABADIE, Jöel, F-25000 Besançon (FR); CHAILLET, Nicolas, F-25870 Bonnay (FR); LEXCELLENT, Christian, F-25002 Besançon (FR)
(74) Mandataire: Bredema
(86) Numéro de dépôt international: PCT/FR2004/000072
(87) Numéro de publication internationale: WO 2004/066831

(56) Documents cités:
- US-A- 5 624 380
- PATENT ABSTRACTS OF JAPAN vol. 2000, no. 08, 6 octobre 2000 (2000-10-06) & JP 2000 135288 A (TOHOKU DENSHI SANGYO KK;MARUYAMA SHIGENAO), 16 mai 2000 (2000-05-16) cité dans la demande
- ABADIE J ET AL: "An integrated shape memory alloy micro-actuator controlled by thermoelectric effect" SENSORS AND ACTUATORS A, ELSEVIER SEQUOIA S.A., LAUSANNE, CH, vol. 99, no. 3, 5 juin 2002 (2002-06-05), pages 297-303, XP004361696 ISSN: 0924-4247
- PATENT ABSTRACTS OF JAPAN vol. 2000, no. 21, 3 août 2001 (2001-08-03) & JP 2001 112796 A (MARUYAMA SHIGENAO;TOHOKU DENSHI SANGYO KK), 24 avril 2001 (2001-04-24)

## Description

La présente invention concerne une structure longitudinale orientable et son procédé de fabrication, ladite structure comprenant des actionneurs substantiellement longitudinaux en alliage à mémoire de forme (AMF), des éléments à effet Peltier dopés N et P ainsi que des moyens de commande électrique.

Une telle structure est notamment destinée aux endoscopes, qu'ils soient utilisés dans le domaine médical ou dans le domaine industriel.

Les endoscopes sont des systèmes optiques permettant l'exploration visuelle de zones inaccessibles. Ils comportent notamment un corps flexible de longueur variable destiné à être introduit dans la zone à contrôler et à l'extrémité duquel est monté un système de vision. L'extrémité de la tête de l'endoscope est articulée de manière à pointer le système de vision dans plusieurs directions, permettant ainsi de visualiser dans la zone à contrôler.

L'art antérieur connaît déjà de nombreuses solutions permettant l'orientation de têtes d'endoscopes.

Outre les systèmes classiques de commande mécanique par l'intermédiaire de câbles en acier qui relient la tête de l'endoscope à l'organe de commande de l'opérateur, l'art antérieur connaît également des solutions basées en particulier sur l'utilisation d'alliages à mémoire de forme.

Il est proposé, dans le brevet américain US5624380, un manipulateur à plusieurs degrés de liberté présentant une pluralité de parties flexibles pouvant être sélectivement fléchies.

Ledit manipulateur est constitué d'un tube flexible comportant :
- une pluralité de parties flexibles disposées en rangées,
- une pluralité d'actionneurs en AMF,
- des moyens de transmission d'énergie (tels que fils conducteurs) aux actionneurs, s'étendant le long du tube flexible,
- des éléments fournissant l'énergie, disposés en série entre lesdits moyens de transmission d'énergie et lesdits actionneurs, afin de contrôler l'énergie fournie aux actionneurs par lesdits moyens de transmission d'énergie.

Selon un mode de réalisation de ce dispositif connu, le manipulateur comporte une pluralité de parties flexibles sensiblement cylindriques disposées selon une direction axiale. Une paire d'éléments déformables thermiquement, chacun étant constitué d'une lame en AMF, est disposée entre les parties flexibles avant et les parties flexibles arrières adjacentes pour courber lesdites parties flexibles.

Il est également proposé dans la demande de brevet japonais JP2000135288 un élément mobile cylindrique pour endoscope ou cathéter, ledit élément cylindrique pouvant être fléchi ou tourné dans une direction demandée avec une vitesse de réponse rapide.

Pour ce faire, ledit élément cylindrique comporte une pluralité d'éléments en AMF ainsi que des éléments Peltier dopés P et dopés N. Ces éléments sont agencés alternativement et sont maintenus entre des anneaux plastiques présentant une cavité centrale.

Les éléments Peltier dopés P et dopés N sont connectés en séries par des électrodes de telle sorte que les parties générant la chaleur et les parties absorbant la chaleur sont dans la même position. Les éléments sont courbés dans une direction demandée par l'application sélectivement de courants aux éléments Peltier.

Les systèmes précédemment décrits présentent cependant de nombreux inconvénients.

En ce qui concerne les systèmes classiques mécaniques, ces derniers présentent l'inconvénient de générer des frottements importants entre les câbles de commande et la gaine de l'endoscope, limitant de ce fait la longueur utile d'exploration.

En outre, le contrôle local entre chaque articulation est impossible, engendrant de ce fait un risque de contact avec certaines zones de l'environnement exploré, risques particulièrement préjudiciables dans le cadre d'explorations fonctionnelles médicales.

Concernant les systèmes développés dans la demande de brevet japonais ou le brevet américain cités ci-dessus, ces derniers présentent également certains désavantages.

En particulier, les actionneurs en AMF utilisés sont généralement sous la forme de fils. Or, de tels fils présentent, en fonction de la température, des allongements relativement restreints. Pour obtenir des actionneurs produisant des déplacements suffisants, il est alors nécessaire d'avoir des fils relativement longs.

En outre, de tels systèmes présentent l'inconvénient de conduire à des endoscopes relativement encombrants du fait de la nature des actionneurs et de leurs dispositions dans l'endoscope. De tels actionneurs limitent en effet généralement l'espace interne de l'endoscope ou augmentent le diamètre externe de celui-ci.

L'article intitulé "An integrated shape memory alloy micro-actuator controlled by thermoelectric effect" de J. Abadie, N. Chaillet , C. Lexcellent, et publié en 2002, décrit un micro-actionneur dont la flexion est obtenue en utilisant l'effet thermoélectrique. II comprend deux éléments à effet Peltier dopés P et N reliés entre eux par une lame en FMA dont la température, et donc la flexion, est contrôlée par effet Peltier.

La présente invention entend remédier aux inconvénients de l'art antérieur en proposant une structure orientable, fonctionnant à partir d'une simple commande électrique, et permettant d'obtenir des endoscopes comportant un nombre limité de pièces nécessaires au fonctionnement ainsi qu'un diamètre fortement réduit pour former des endoscopes à faible encombrement tout en garantissant une bonne résolution de positionnement de la tête.

Pour ce faire, la présente invention est du type décrit ci-dessus et elle est remarquable, dans son acception la plus large, en ce que lesdits actionneurs sont agencés par paire et disposés parallèlement de manière antagoniste, c'est à dire en opposition par rapport à leur forme mémorisée respective, chaque actionneur étant en contact sensiblement à ses extrémités avec respectivement un élément à effet Peltier dopé N et un élément à effet Peltier dopé P, l'ensemble étant monté en série avec les moyens de commande électrique. Pour former un circuit thermoélectrique de façon à ce que, pour un sens fixé de courant appliqué, un des actionneurs de ladite paire va s'échauffer et subira une flexion vers sa forme mémorisée, tandis que l'actionneur disposé de manière dite antagoniste va refroidir et subira une flexion opposée à sa forme mémorisée.

De préférence, lesdits actionneurs sont des lames, de préférence monoblocs.

L'assemblage des actionneurs avec les éléments à effet Peltier est réalisé préférentiellement par soudure.

Avantageusement, chaque élément à effet Peltier dopé N et dopé P est en contact avec un élément partiellement annulaire conducteur, lequel est de préférence en cuivre. La mise en contact est préférentiellement réalisée par soudure.

Avantageusement, lesdits actionneurs, associés aux éléments à effet Peltier, sont disposés de manière diamétralement opposée par rapport à l'axe longitudinal de la structure.

Avantageusement, lesdits actionneurs sont constitués d'alliage de nickel titane (NiTi), et lesdits éléments à effet Peltier de Tellure de Bismuth.

En outre, ladite structure comporte de la résine époxy recouvrant lesdits éléments à effet Peltier, y compris les jonctions thermoélectriques avec lesdits actionneurs, ceci afin de renforcer lesdits éléments à effet Peltier.

La présente invention se rapporte également à un endoscope comprenant un corps longitudinal présentant à son extrémité distale un système de vision, caractérisé en ce qu'au moins une partie du corps longitudinal est formée avec au moins une structure longitudinale orientable décrite précédemment.

De préférence, au moins une partie du corps longitudinal dudit endoscope est formée d'une pluralité de structures longitudinales orientables, lesdites structures étant empilées les unes sur les autres de telle sorte que l'élément conducteur d'une desdites structures portant les éléments dopés N est adjacent avec l'élément conducteur portant les éléments à effet Peltier dopés P de la structure précédente.

Avantageusement, les actionneurs d'une structure au moins présentent avec les actionneurs d'une structure précédente et/ou suivante des déformations dans des directions différentes.

La présente invention se rapporte également au procédé de fabrication d'une structure longitudinale orientable telles que décrite ci-dessus et dont les actionneurs en AMF sont des lames. Ledit procédé comporte successivement :
- une étape de préparation des actionneurs en AMF consistant à découper des lames présentant une forme courbée dans une plaque en AMF, de préférence en NiTi, ladite forme courbée des lames correspondant à une forme dite mémorisée ;
- une étape de refroidissement desdites lames jusqu'à l'obtention de lames sensiblement droites ;
- une étape d'assemblage desdites lames obtenues lors de l'étape précédente aux dits éléments à effet Peltier, ladite étape d'assemblage consistant à intégrer lesdites lames entre lesdits éléments à effet Peltier dopés N et P.

Ledit procédé de fabrication requiert en outre une étape d'assemblage desdits éléments à effet Peltier à des éléments annulaires conducteurs, de préférence en cuivre, ainsi qu'une étape de coulage de résine pour recouvrir lesdits éléments à effet Peltier, y compris les jonctions thermoélectriques avec lesdits actionneurs.

De préférence, l'étape d'assemblage desdites lames et desdits éléments conducteurs aux dits éléments à effet Peltier consiste en une opération de soudage.

On comprendra mieux l'invention à l'aide de la description, faite ci-après à titre purement explicatif, d'un mode de réalisation de l'invention, en référence aux figures annexées :
- la figure 1 illustre une vue de dessus d'une structure longitudinale selon l'invention ;
- la figure 2 illustre une vue en coupe de la structure de la figure 1 selon l'axe AA' ;
- la figure 3 illustre une vue en coupe de la structure de la figure 1 selon l'axe BB' ;
- la figure 4 illustre une vue en coupe de la structure de la figure 1 selon l'axe BB', ladite structure présentant un mouvement de flexion ;
- la figure 5 illustre une vue schématisée d'un corps longitudinal d'un endoscope formé d'un empilement de structures selon l'invention.

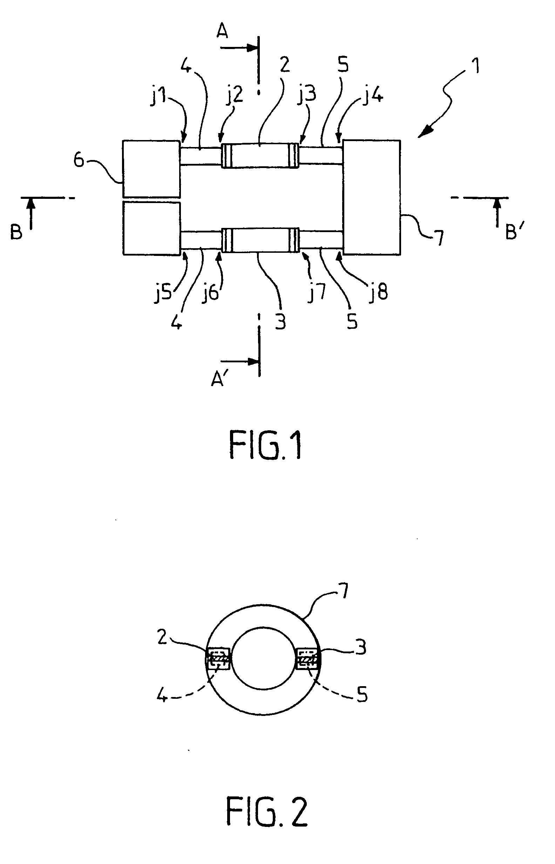

La structure longitudinale orientable (1) selon l'invention, illustrée figure 1, comporte deux lames (2, 3) en alliage à mémoire de forme (AMF).

Lesdites lames (2, 3) en AMF sont disposées respectivement entre des éléments à effet Peltier dopés N (4) et P (5), de sorte que les extrémités de chaque lame (2, 3) sont en contact respectivement avec l'une des extrémités d'un élément à effet Peltier dopé N (4) et l'une des extrémités d'un élément à effet Peltier dopé P (5).

Deux éléments annulaires conducteurs (6, 7) sont respectivement disposés de part et d'autre des extrémités libres desdits éléments dopés N et P (4, 5).

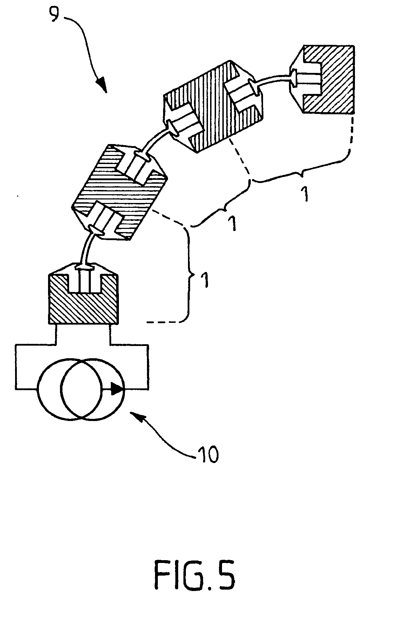

Pour un parcours de courant électrique correct, les éléments annulaires (6, 7) sont de préférence partagés en deux demi-éléments annulaires isolés électriquement. Sur la figure 1, il a été représenté une structure (1) comportant deux demi-éléments annulaires (6) et un élément annulaire (7) . Pour d'autres applications, nécessitant notamment l'empilage d'une pluralité de structures (1) comme représenté sur la figure 5, ladite structure comportera préférentiellement quatre demi-éléments annulaires (6, 7).

On obtient ainsi un circuit thermoélectrique formé d'un empilement du premier élément conducteur (6), mis en contact avec deux éléments à effet Peltier dopés N (4), lesquels sont fixées respectivement à l'une des extrémités d'une desdites lames (2, 3), l'autre extrémité desdites lames (2, 3) étant connectée respectivement aux deux éléments à effet Peltier dopés P (5) également en contact avec le deuxième élément conducteur (7).

De préférence, lesdites lames (2,3), associées aux éléments à effet Peltier (4, 5), sont disposées de manière diamétralement opposée par rapport à l'axe longitudinal de ladite structure (1), comme illustré sur la figure 2.

Avantageusement, lesdites lames en AMF (2, 3) sont des lames monoblocs constituées à base d'alliage de nickel titane.

Avantageusement, lesdits éléments à effet Peltier (4, 5) sont constitués de Tellure de Bismuth (TeBi), et lesdits éléments conducteurs (6, 7) de cuivre.

Bien que non représenté sur la figure 1, ladite structure (1) est connectée à des moyens de commande électrique, du type générateur de courant (10) comme illustré sur la figure 5.

Le principe de fabrication de la structure (1) selon l'invention se fait comme suit.

Les lames (2, 3) sont découpées soit à haute température T, soit à température ambiante Ta après un refroidissement sans contrainte, dans une plaque en NiTi selon une forme courbée. La forme initiale de ces lames (2, 3) correspond à une forme dite mémorisée. Ainsi, si la température est diminuée jusqu'à la température ambiante Ta, il est possible de tordre lesdites lames (2, 3) jusqu'à l'obtention d'une courbure inverse à la courbure relative à la forme mémorisée. Pour redonner aux dites lames (2, 3) leur forme mémorisée, il suffit de les chauffer à nouveau jusqu'à la température T.

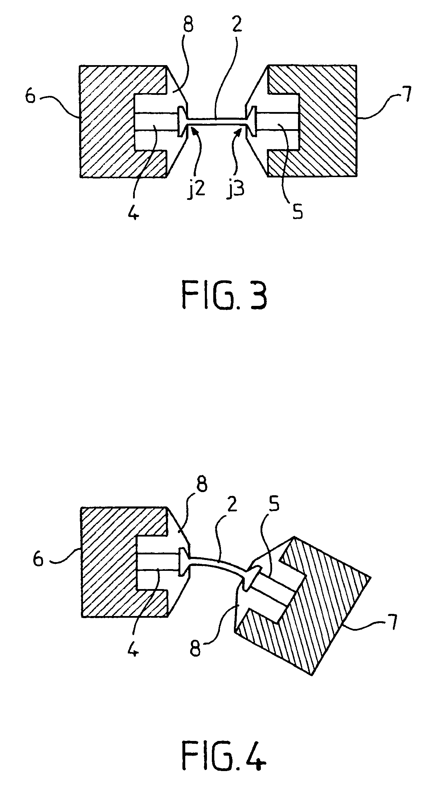

Une fois découpés, lesdites lames (2, 3) sont refroidies puis déformées jusqu'à l'obtention d'une forme sensiblement droite (cf. figure 3).

Lesdites lames (2, 3), ainsi tordues pour obtenir des formes sensiblement droites, sont alors couplées aux éléments à effet Peltier (4, 5), réalisant ainsi des jonctions thermoélectriques (j2, j3, j6, j7). Plus particulièrement, chaque lame (2, 3) est intégrée entre un élément à effet Peltier dopé N (4) et un élément à effet Peltier dopé P (5), en prenant bien soin de monter lesdites lames (2, 3) en opposition par rapport à leur forme mémorisée respective. Ainsi, pour un sens du courant fixé, l'opérateur dirigera ladite structure (1) selon la direction souhaitée : une des lames (celle qui chauffe) (2, 3) provoquera la flexion du module grâce à l'effet mémoire, pendant que l'autre (celle qui refroidit) (3, 2), subira une déformation dans le sens opposé à sa forme mémorisée,

Préalablement, la fabrication de ladite structure (1) comporte une étape d'assemblage desdits éléments à effet Peltier (4, 5) aux dits des éléments conducteurs (6, 7), réalisant ainsi des jonctions thermoélectriques (j1, j4, j5, j8).

La structure (1) obtenue forme ainsi un circuit thermoélectrique, lequel transforme l'énergie électrique transmise par lesdits moyens de commande électrique en énergie thermique qui est alors communiquée aux dites lames (2, 3). Lesdites lames (1, 2), faisant partie intégrante du système de conversion thermoélectrique, convertissent alors l'énergie thermique reçue en énergie mécanique, générant le mouvement de flexion de la structure (1).

La figure 3 illustre une vue en coupe de ladite structure (1) selon l'axe BB' de la figure 1.

Sur cette figure, est représentée la structure (1) comprenant, sur une partie, de la résine époxy (8). Cette résine (8) est destinée à renforcer lesdits éléments à effet Peltier (4, 5) ainsi que les jonctions thermoélectriques (j1 à j8) constituant ladite structure (1). En effet, le mouvement de flexion est transmis, comme l'illustre la figure 4, entre les lames (2, 3) en AMF et les éléments conducteurs (6, 7), lesdits éléments à effet Peltier (4, 5) subissant de ce fait une charge importante. L'adjonction de résine époxy permet donc de renforcer lesdits éléments à effet Peltier (4, 5).

Par l'utilisation desdits moyens de commande électrique auxquels ladite structure (1) est reliée, un courant est transmis dans le circuit thermoélectrique, lequel fourni l'énergie thermique aux dites lames (2, 3), lesquelles, en réponse, génèrent à ladite structure (1) un mouvement de flexion (figure 4).

Selon que le courant appliqué à ladite structure (1) soit positif ou négatif, le mouvement de flexion se fera dans un sens ou dans un autre. Ainsi, lors de l'application d'un courant positif (négatif) à ladite structure (1), l'une des lames va refroidir (s'échauffer), subissant une flexion opposée à sa forme mémorisée (flexion vers sa forme mémorisée), tandis que l'autre lame va s'échauffer (refroidir), subissant alors une flexion vers sa forme mémorisée (flexion opposée à sa forme mémorisée).

Lesdites lames (2, 3) ayant été disposées entre lesdits éléments à effet Peltier (4, 5) lors de l'étape d'assemblage de manière à subir dans un même sens des déformations opposées, la lame (2, 3) qui, pour un sens du courant fixé, s'échauffe, va provoquer la flexion de ladite structure (1) grâce à l'effet mémoire, pendant que l'autre lame (3, 2), qui en conséquence refroidit, subit une déformation de type réorientation de la martensite. Le sens de la flexion de ladite structure (1) est donc donné par la lame (2, 3) qui s'échauffe jouant le rôle de « lame motrice », la deuxième lame (3, 2) jouant le rôle de « lame esclave ».

La figure 5 illustre une vue schématisée d'un corps longitudinal (9) d'un endoscope formé par exemple d'un empilement de trois structures longitudinales orientables (1), chacune desdites structures étant identique et simplement connectée électriquement à la précédente ou la suivante, avec une possibilité d'orientation autour de l'axe longitudinal.

L'empilement est réalisé de telle sorte que l'élément conducteur (6) de ladite structure (1) portant les éléments dopés N est adjacent avec l'élément conducteur (7) portant les éléments à effet Peltier dopés P de la structure précédente.

Dans un mode de réalisation avantageux de l'invention, les lames en AMF d'une première structure (1) présentent avec les lames en AMF d'une seconde structure (1) précédente et/ou suivante des déformations dans des directions différentes.

Bien entendu, les lames en AMF d'une structure (1) peuvent présenter avec les lames en AMF d'une autre structure (1) précédente et/ou suivante des déformations dans des directions identiques.

L'invention est décrite dans ce qui précède à titre d'exemple. Il est entendu que l'homme du métier est à même de réaliser différentes variantes de l'invention sans pour autant sortir du cadre du brevet.

## Revendications

1. Structure longitudinale orientable (1) comprenant des actionneurs substantiellement longitudinaux en alliage à mémoire de forme, des éléments à effet Peltier dopés N et P (4, 5) ainsi que des moyens de commande électrique, lesdits actionneurs étant agencés par paire et disposés parallèlement de manière antagoniste, c'est-à-dire en opposition par rapport à leur forme mémorisée respective, chaque actionneur étant en contact sensiblement à ses extrémités avec respectivement un élément à effet Peltier dopé N (4) et un élément à effet Peltier dopé P (5), l'ensemble étant monté en série avec les moyens de commande électrique pour former un circuit thermoélectrique de façon à ce que, pour un sens fixé de courant appliqué, un des actionneurs de ladite paire va s'échauffer et subira une flexion vers sa forme mémorisée, tandis que l'actionneur disposé de manière dite antagoniste va refroidir et subira une flexion opposée à sa forme mémorisée.

2. Structure longitudinale orientable (1) selon la revendication 1, **caractérisée en ce que** lesdits actionneurs sont des lames (2, 3), de préférence monoblocs.

3. Structure longitudinale orientable selon la revendication 1 ou selon la revendication 2, **caractérisée en ce que** chaque élément à effet Peltier dopé N (4) et dopé P (5) est en contact avec un élément partiellement annulaire conducteur (6, 7), de préférence en cuivre.

4. Structure longitudinale orientable selon la revendication 3, **caractérisée en ce que** chaque élément à effet Peltier dopé N (4) et dopé P (5) est soudé audit élément conducteur (6, 7).

5. Structure longitudinale orientable (1) selon l'une quelconque des revendications 1 à 4, **caractérisée en ce que** lesdits actionneurs, associés aux éléments à effet Peltier (4, 5), sont disposés de manière diamétralement opposée par rapport à l'axe longitudinal de la structure (1).

6. Structure longitudinale orientable (1) selon l'une quelconque des revendications précédentes, **caractérisée en ce que** lesdits actionneurs sont soudés aux dits éléments à effet Peltier dopé N et P (4, 5).

7. Structure longitudinale orientable (1) selon l'une quelconque des revendications précédentes, **caractérisée en ce que** lesdits actionneurs sont constitués d'alliage de nickel titane(NiTi).

8. Structure longitudinale orientable (1) selon l'une quelconque des revendications précédentes, **caractérisée en ce que** lesdits éléments à effet Peltier (4, 5) sont constitués de Tellure de Bismuth.

9. Structure longitudinale orientable (1) selon l'une quelconque des revendications précédentes, **caractérisée en ce que** ladite structure (1) comporte en outre de la résine époxy (8) recouvrant lesdits éléments à effet Peltier (4, 5), y compris les jonctions thermoélectriques (j2, j3, j6, j7) avec lesdits actionneurs.

10. Endoscope comprenant un corps longitudinal (9) présentant à son extrémité distale un système de vision, **caractérisé en ce qu'**au moins une partie du corps longitudinal (9) est formée avec au moins une structure longitudinale orientable (1) selon l'une quelconque des revendications précédentes.

11. Endoscope selon la revendication précédente, **caractérisé en ce qu'**au moins une partie du corps longitudinal (9) est formée d'une pluralité de structures orientables (1), lesdites structures (1) étant empilées les unes sur les autres de telle sorte que l'élément conducteur d'une desdites structures (1) portant les éléments dopés N est adjacent avec l'élément conducteur portant les éléments à effet Peltier dopés P de la structure (1) précédente.

12. Endoscope selon la revendication 10 ou la revendication 11, **caractérisé en ce que** les actionneurs d'une structure (1) au moins présentent avec les actionneurs d'une structure (1) précédente et/ou suivante des déformations dans des directions différentes.

13. Procédé de fabrication d'une structure longitudinale orientable (1) selon l'une quelconque des revendications 2 à 9, **caractérisé en ce que** ledit procédé comporte successivement :
- une étape de préparation des actionneurs en AMF consistant à découper des lames (2, 3) présentant une forme courbée dans une plaque en AMF, de préférence en NiTi, ladite forme courbée des lames (2, 3) correspondant à une forme dite mémorisée ;
- une étape de refroidissement desdites lames (2, 3) jusqu'à l'obtention de lames (2, 3) sensiblement droites ;
- une étape d'assemblage desdites lames (2, 3) obtenues lors de l'étape précédente aux dits éléments à effet Peltier (4, 5), ladite étape d'assemblage consistant à intégrer lesdites lames (2, 3) entre lesdits éléments à effet Peltier dopés N et P (4, 5).

14. Procédé de fabrication selon la revendication précédente, **caractérisé en ce qu'**il comporte en outre une étape d'assemblage desdits éléments à effet Peltier (4, 5) à des éléments partiellement annulaires conducteurs (6, 7), de préférence en cuivre.

15. Procédé de fabrication selon la revendication 13 ou la revendication 14, **caractérisé en ce que** les étapes d'assemblage consistent en une opération de soudage.

16. Procédé de fabrication selon l'une quelconque des revendications 13 à 15, **caractérisé en ce que** ledit procédé comporte en outre une étape de coulage de résine (8) pour recouvrir lesdits éléments à effet Peltier (4, 5), y compris les jonctions thermoélectriques (j2, j3, j6, j7) avec lesdits actionneurs.

## Claims

1. An orientable longitudinal structure (1) comprising substantially longitudinal actuators made of a shape memory alloy, n- and p-doped elements (4, 5) using Peltier effect, as well as electric control means, said actuators being arranged in pairs and positioned in parallel in an antagonist way, i.e. opposite each other with respect to their respective memorised shape, each actuator being in contact, substantially at the ends thereof, with an n-doped element using Peltier effect (4) and a p-doped element using Peltier effect (5) respectively, the assembly being mounted in series with the electric control means to form a thermoelectric circuit so that, for a fixed direction of the applied current, one of the actuators of said pair will heat and will undergo a flexion towards its memorized shape, whereas the actuator positioned in the so-called antagonist way will cool and undergo a flexion opposite its memorized shape.

2. An orientable longitudinal structure (1) according to claim 1, **characterised in that** said actuators are blades (2, 3) preferably one-piece blades.

3. An orientable longitudinal structure according to claim 1 or according to claim 2, **characterised in that** each n-doped (4) and p-doped (5) element using Peltier effect is in contact with a partially annular conducting element (6, 7), preferably made of copper.

4. An orientable longitudinal structure according to claim 3, **characterised in that** each n-doped (4) and p-doped (5) element using Peltier effect is welded to said conducting element (6, 7).

5. An orientable longitudinal structure (1) according to any one of claims 1 to 4, **characterised in that** said actuators, associated with the elements using Peltier effect (4, 5) are positioned in a diametrally opposite way with respect to the longitudinal axis of the structure (1).

6. An orientable longitudinal structure (1) according to any one of the preceding claims, **characterised in that** said actuators are welded to said n- and p-doped elements using Peltier effect (4, 5).

7. An orientable longitudinal structure (1) according to any one of the preceding claims, **characterised in that** said actuators are composed of a nickel titanium (NiTi) alloy.

8. An orientable longitudinal structure (1) according to any one of the preceding claims, **characterised in that** said elements using Peltier effect (4, 5) are composed of Bismuth Telluride.

9. An orientable longitudinal structure (1) according to any one of the preceding claims, **characterised in that** said structure (1) further comprises epoxy resin (8) covering said elements using Peltier effect (4, 5) including the thermoelectric junctions (j2, j3, j6, j7) with said actuators.

10. An endoscope comprising a longitudinal body (9) having a viewing system at the distal end thereof, **characterised in that** at least a part on the longitudinal body (9) is formed with at least one orientable longitudinal structure (1) according to any one of the preceding claims.

11. An endoscope according to the preceding claim, **characterised in that** at least one part of the longitudinal body (9) is formed of a plurality of orientable structures (1), said structures (1) being stacked upon each other so that the conducting element of one of said structures (1) carrying the n-doped elements is adjacent to the conducting element carrying the p-doped elements using Peltier effect of the preceding structure (1).

12. An endoscope according to claim 10 or claim 11, **characterised in that** the actuators of a structure (1) show deformations in different directions at least with the actuators of a preceding and/or following structure (1).

13. A method for producing an orientable longitudinal structure (1) according to any one of claims 2 to 9, **characterised in that** said method successively includes:
- a step of preparation of actuators made of SMA consisting in cutting blades (2, 3) having a curved shape in a plate made of SMA, preferably made of NiTi, said curved shape of the blades (2, 3) corresponding to a so-called memorized shape,
- a step of cooling said blades (2, 3) until substantially straight blades (2, 3) are obtained;
- a step of assembling said blades (2, 3) obtained during the preceding step with said elements using Peltier effect (4, 5), said assembling step consisting in integrating said blades (2, 3) between said n-doped and p-doped elements using Peltier effect (4, 5).

14. A method of production according to the preceding claim, **characterised in that** it further includes a step of assembling said elements using Peltier effect (4, 5) to partially annular conducting elements (6, 7), preferably made of copper.

15. A method of production according to claim 13 or claim 14, **characterised in that** the assembling steps consist in a welding operation.

16. A method of production according to any one of claims 13 to 15, **characterised in that** said method further includes a step of casting resin (8) for covering said elements using Peltier effect (4, 5), inclusive of the thermoelectric junctions (j2, j3, j6, j7) with said actuators.

## Patentansprüche

1. Drehbare Längsstruktur (1) mit im Wesentlichen Längsbetätigungsorganen aus Formspeicherlegierung, N- und P-gedopten Peltier-Elementen (4, 5) sowie elektrischen Steuermitteln, wobei die genannten Betätigungsorgane paarweise und parallel entgegenwirkend angeordnet sind, d. h. entgegen ihrer jeweiligen gespeicherten Form, wobei jedes Betätigungsorgan etwa an seinen Enden jeweils in Kontakt steht mit einem N-gedopten Peltier-Element (4) und einem P-gedopten Peltier-Element (5), wobei die Einheit in Reihe montiert ist mit den elektrischen Steuermitteln, um einen thermoelektrischen Kreislauf zu bilden, so dass für eine festgelegte Richtung des angelegten Stroms eines der Betätigungsorgane des genannten Paars sich erwärmt und eine Flexion in Richtung seiner gespeicherten Form erfährt, wobei sich das andere, entgegenwirkend angeordnete Betätigungsorgan abkühlt und eine Flexion in die seiner gespeicherten Form entgegengesetzte Richtung erfährt.

2. Drehbare Längsstruktur (1) gemäß Anspruch 1, **dadurch gekennzeichnet, dass** die genannten Betätigungsorgane bevorzugt einteilige Lamellen (2, 3) sind.

3. Drehbare Längsstruktur gemäß Anspruch 1 oder Anspruch 2, **dadurch gekennzeichnet, dass** jedes N-gedopte und P-gedopte Peltier-Element (4, 5) mit einem teilweise ringförmigen leitenden Element (6, 7), bevorzugt aus Kupfer, in Verbindung steht.

4. Drehbare Längsstruktur gemäß Anspruch 3, **dadurch gekennzeichnet, dass** jedes N-gedopte und P-gedopte Peltier-Element (4, 5) am genannten leitenden Element (6, 7) angeschweißt ist.

5. Längsstruktur (1) gemäß einem der Ansprüche 1 bis 4, **dadurch gekennzeichnet, dass** die genannten, mit den Peltier-Elementen (4, 5) verbundenen Betätigungsorgane in Bezug auf die Längsachse der Struktur (1) diametral gegenüberliegend angeordnet sind.

6. Drehbare Längsstruktur (1) gemäß einem der vorangehenden Ansprüche, **dadurch gekennzeichnet, dass** die genannten Betätigungsorgane an den genannten N-gedopten und P-gedopten Peltier-Elementen (4, 5) angeschweißt sind.

7. Drehbare Längsstruktur (1) gemäß einem der vorangehenden Ansprüche, **dadurch gekennzeichnet, dass** die genannten Betätigungsorgane aus Nickel-Titan-Legierung (NiTi) bestehen.

8. Drehbare Längsstruktur (1) gemäß einem der vorangehenden Ansprüche, **dadurch gekennzeichnet, dass** die genannten Peltier-Elemente (4, 5) aus Wismut-Tellur bestehen.

9. Drehbare Längsstruktur (1) gemäß einem der vorangehenden Ansprüche, **dadurch gekennzeichnet, dass** die genannte Struktur (1) ferner Epoxydharz (8) umfasst, das die genannten Peltier-Elemente (4, 5) einschließlich die thermoelektrischen Verbindungen (j2, j3, j6, j7) mit den genannten Betätigungsorganen bedeckt.

10. Endoskop mit einem Längskörper (9), der an seinem distalen Ende ein Sichtsystem aufweist, **dadurch gekennzeichnet, dass** mindestens ein Teil des Längskörpers (9) mit mindestens einer drehbaren Längsstruktur (1) gemäß einem der vorangehenden Ansprüche gebildet ist.

11. Endoskop gemäß dem vorangehenden Anspruch, **dadurch gekennzeichnet, dass** mindestens ein Teil des Längskörpers (9) aus einer Mehrzahl von drehbaren Strukturen (1) gebildet ist, wobei die genannten Strukturen (1) aufeinander gestapelt sind, so dass das leitende Element einer der genannten Strukturen (1), das die N-gedopten Elemente trägt, an das leitende Element angrenzt, das die P-gedopten Peltier-Elemente der vorherigen Struktur (1) trägt.

12. Endoskop gemäß Anspruch 10 oder Anspruch 11, **dadurch gekennzeichnet, dass** die Betätigungsorgane mindestens einer Struktur (1) mit den Betätigungsorganen einer vorherigen und/oder nachfolgenden Struktur (1) Verformungen in verschiedenen Richtungen aufweisen.

13. Herstellungsverfahren einer drehbaren Längsstruktur (1) gemäß einem der Ansprüche 2 bis 9, **dadurch gekennzeichnet, dass** das genannte Verfahren die aufeinanderfolgenden Schritte umfasst:
- einen Vorbereitungsschritt der Betätigungsorgane aus AMF, der darin besteht, aus einer Platte aus AMF, bevorzugt aus NiTi, gekrümmte Lamellen (2, 3) auszustanzen, wobei die genannte gekrümmte Form der Lamellen (2, 3) einer so genannten gespeicherten Form entsprechen;
- einen Kühlungsschritt der genannten Lamellen (2, 3), bis zum Erhalt von etwa geraden Lamellen (2, 3);
- einen Zusammensetzungsschritt der genannten beim vorherigen Schritt erhaltenen Lamellen (2, 3) mit den Peltier-Elementen (4, 5), wobei der genannte Zusammensetzungsschritt darin besteht, die genannten Lamellen (2, 3) zwischen den genannten N- und P-gedopten Peltier-Elementen (4, 5) einzufügen.

14. Herstellungsverfahren gemäß dem vorangehenden Anspruch, **dadurch gekennzeichnet, dass** es ferner einen Zusammensetzungsschritt der genannten Peltier-Elemente (4, 5) mit den teilweise ringförmigen, leitenden, bevorzugt aus Kupfer bestehenden Elementen (6, 7) umfasst.

15. Herstellungsverfahren gemäß Anspruch 13 oder Anspruch 14, **dadurch gekennzeichnet, dass** die Zusammensetzungsschritte aus einer Schweißoperation bestehen.

16. Herstellungsverfahren gemäß einem der Ansprüche 13 bis 15, **dadurch gekennzeichnet, dass** das genannte Verfahren ferner einen Harzverguss-Schritt (8) umfasst, um die genannten Peltier-Elemente (4, 5) einschließlich die elektrothermischen Verbindungen (j2, j3, j6, j7) mit den genannten Betätigungsorganen zu bedecken.
